# EUROPEAN PATENT SPECIFICATION

(11) **EP 2 923 679 B1**
(45) Date of publication and mention of the grant of the patent: **01.11.2017**
(21) Application number: 14382105.6
(22) Date of filing: 26.03.2014
(51) Int. Cl.: A61F 5/08

(54) **Nasal device**
Nasenvorrichtung
Dispositif nasal

(43) Date of publication of application: 30.09.2015
(73) Proprietor: Martin Prieto, Antonio, 1203 Ginebra (CH)
(72) Inventor: Martin Prieto, Antonio, 1203 Ginebra (CH)
(74) Representative: Temiño Ceniceros, Ignacio

(56) References cited:
- DE-U1- 29 616 121
- DE-U1- 29 820 401
- US-A- 1 597 331
- US-A- 4 778 466
- US-A1- 2003 144 684
- US-A1- 2011 218 451

## Description

### FIELD OF THE INVENTION

The present invention belongs to the field of nasal devices for modifying the nasal airflow. More specifically, the invention relates to a nasal device for modifying the nasal airflow by separating the body tissues, and by creating expiratory positive airway pressure (EPAP) in a subject's airway.

### BACKGROUND OF THE INVENTION

Currently, the field of nasal devices for modifying the nasal airflow comprises a wide variety of business solutions, which can be mainly divided into two groups according to the way in which they modify said flow.

A first group, in which some of the embodiments of the invention proposed herein can be classified, includes the devices which modify the nasal airflow by separating the body tissues of the nasal anatomy. This separation of tissues increases the surface of the flow section of the airway and prevents the possible collapse of the same. Therefore, these devices improve the amount and flow of breathed air, with the consequent therapeutic benefits. This group is mainly aimed at the treatment of the "Upper Airway Resistance Syndrome" (or UARS), derived often from dysfunctions in the areas of the nasal valve and vestibule. The cited group includes devices called nasal dilators or spreaders, such as adhesive strips, cannulas, clips, wires, etc.

The second group, technically more complex, includes devices that modify the nasal airflow by creating positive pressure inside the airways. Such pressure counteracts the excessive negative pressure generated by the UARS inside said airways, which can make them collapse, as in the case of patients with "sleep apnea-hypopnea syndrome" (or SAHS). In this way, the positive pressure frees up and expands the airways, facilitating and improving the nasal airflow. This second group of devices is, in general, more focused on the treatment of SAHS and, more specifically, on the treatment of "obstructive" sleep apnea-hypopnea.

Depending on the way in which the inventions belonging to this second group generate positive airway pressure, it is possible to differentiate two sub-groups:
- A first subgroup, which creates positive airway pressure by injecting a continuous stream of air into the interior of the nasal airway, by means of a nasal or naso-buccal mask and an external air pump. Normally, the devices belonging to this subgroup need a source of electrical power to supply the air pump. This subgroup includes the devices based on the technology known as "continuous positive airway pressure" (or CPAP), which is one of those used for the treatment of SAHS.
- A second subgroup, in which some of the embodiments of the invention proposed herein can also be classified, of devices which create positive airway pressure using airflow resistances located in the upper airways, which resist the exit of air during the expiratory phase. This pressure is referred to as positive end expiratory pressure (PEEP) or expiratory positive airway pressure (EPAP).

Despite the plurality of existing proposals in the area of nasal devices for the treatment of SAHS, there is a persisting problem in that many patients do not tolerate the standard CPAP-based treatments for this type of syndrome, due to side effects such as swelling of the stomach and abdominal area, headaches caused by the use of the naso-buccal mask, irritation of the mucous membranes as a result of the excessive air transit, weakening of the diaphragm in the inspiratory phase, and difficulties generated by the limitation of positions that the user can take while sleeping. Such cases represent around 50% of the total patients, a very high percentage.

In addition, other systems are known in the state of the art, such as the one described in patent application US2003/0144684, which is considered the closest prior art and refers to an adjustable nasal dilator filter to improve nasal air filtration which consists of two symmetric polymer loops formed by a retaining tube. Also, patent US1597331 discloses a nostril expander which consists of a skeleton-like device composed of two members of substantially ellipsoidal shape in general outline joined together by a substantially U-shaped bridge piece whose side portions are connected at their upper ends to intermediate portions of the lower sides of said members. However, both systems provide limited mechanical response capacities and fail at improving the response to the stress forces applied while using the device.

Additionally, many of the known devices are uncomfortable to use, since they necessarily require a power supply, which also complicates their transport during the time of application.

The invention described herein is intended to solve the technical problems above, using a new nasal device based on techniques of nasal dilatation or spreading, optionally in combination with techniques for the creation of positive airway pressure of the EPAP type.

### BRIEF DESCRIPTION OF THE INVENTION

The present invention is based on a new functional logic that uses the physical and geometrical properties of toroidal structures for modifying a subject's nasal airflow, and the objective of which is a device for the treatment of different types of dysfunctions and medical disorders of respiratory and orthodontic type, although it can also be used for non-therapeutic purposes, as for example in sports requiring aerobic power or during intellectual activities in need of high concentration. In addition, the invention can be used complementarily with other known nasal devices or therapies.

To achieve the aforementioned objective, a toroidal nasal device according to the invention comprises, preferably, a toroidal structure to stabilize itself in the subject's nasal cavities, to modify the nasal airflow, and to adapt itself to the variety of existing nasal physiognomies. More specifically, the invention relates to a device according to the independent and dependent claims included in the present document.

In different preferred embodiments of the invention, a toroidal nasal device can be configured to fit, partially, in the subject's nasal cavities and modify its nasal airflow by separating the body tissues, more specifically, the tissues of the nasal anatomy. In some of its variants, the toroidal nasal device can also be configured to modify a subject's nasal airflow, by simultaneously separating body tissues and creating expiratory positive airway pressure (EPAP). This is the case of a variant of the toroidal nasal device that comprises a plurality of airflow resistances connected to its toroidal structure.

All the toroidal nasal devices can be configured with elastic and/or flexible elements, in order to be able to adapt and adjust better to the great variety of existing physiognomies. A toroidal nasal device can modify the nasal airflow, in any of its forms, acting on the external and internal nasal valve simultaneously and without substantially altering the filtering function of the barrier of nose hairs (vibrissae). It can also allow a maximum contact surface between the flow of breathed air and the nasal mucosa, thus improving the quality of the former in terms of the level of humidity and the impurities it contains.

In general, a nasal device according to the invention comprises, preferably:
- a flexible toroidal structure, suitable to be introduced partially into the subject's nasal cavities;
- a closing element connected to the toroidal structure, equipped with means to alter the shape of said flexible toroidal structure, causing it to adopt a new shape comprising at least two lobes of the toroidal structure, each of which can be inserted into a subject's nasal cavity;
and in such a way that, when the device is inserted into the subject's nasal cavities, the closing element is arranged in the lower external region of the nose between the two nostrils and each of the lobes of the toroidal structure presses against the inner surface of the nasal cavities, modifying the subject's nasal airflow.

As mentioned in the previous paragraphs, the toroidal nasal device also includes, optionally, one or more airflow resistances, designed specifically for this type of devices, which are anchored to the toroidal structure (for example, elastic flap valves) to be located inside of the nasal cavities and regulating the nasal airflow through them, creating expiratory positive airway pressure (EPAP). In one of its variants, the toroidal nasal device also includes an adhesive element, optionally replaceable, to attach the closing element at the bottom of a subject's nose, between the two nostrils.

Preferably, the toroidal nasal devices can be installed and removed by the user using only his or her fingers, without any specialized instrument.

The operation of the toroidal nasal device of the invention, including all its variants, essentially depends on the physical and geometrical properties of a toroidal structure (ring-shaped closed surface of revolution) and the closing element that modifies its geometry. One of the properties of the toroidal structures, essential for the functioning of a toroidal nasal device, is to allow, inside its ring geometry, the loop circulation of stresses (torsion, compression, flexion) generated by the application of a force external to it. This characteristic gives the toroidal structure a good mechanical response capacity with flexible materials and slim construction sections. The use of materials with flexible consistencies in the toroidal nasal device, closest to that of the body tissues on which it will act, can improve user comfort during periods of prolonged use.

In the examples described in this document, the toroidal nasal device includes a closure system that modifies the geometry of the toroidal structure, during the periods of use of the device, in order to create an internal stress in the materials of said structure that improves its elastic behavior. This internal tension allows an optimal mechanical behavior of the toroidal structure as well as savings in the materials used. Said closing element is anchored to the toroidal structure, to modify its geometry, providing a patellar anchor, i.e., an anchor that allows the free spinning of the toroidal structure body inside the closing element, in order not to create additional torsional stresses in the structure that may impair its proper operation.

In one of its variants, the closing element of the toroidal nasal device can also be attached temporarily at the bottom of the nose, between the two nostrils, for example, through a support element in clip form. This allows it to collaborate in a supporting manner with the toroidal structure in stabilizing the device in the subject's nasal cavities, preventing possible lateral movements or unwanted handlings of the same, as for example during the sleep phase or during a sporting activity. In another variant of the invention, the closing element can be attached to the nose by means of a fixed or replaceable adhesive element.

The characteristic ring shape of a toroidal structure gives it great flexibility and mechanical responsiveness in the three spatial directions. This allows it to better adapt to the different geometries of the nasal anatomy, to stabilize itself inside these, to separate the nasal tissues by applying a slight pressure along its curvilinear geometry, and to locate the airflow resistances inside the nasal cavities, ahead of or behind the internal nasal valve.

The toroidal nasal device of the invention can modify the subject's nasal airflow simultaneously separating the tissues of the areas of the external nasal valve and the internal nasal valve. Moreover, it has the ability to act on two separate points of the internal nasal valve, one in the upper area and another in the lower area thereof, so that the area of the flow section of the valve increases effectively. In some of its embodiments, the toroidal nasal device can also incorporate one or more airflow resistances designed specifically for this type of device that, once lodged in the nasal cavities, can regulate the flow of air in at least one of its two directions. In different embodiments of the invention, the specific airflow resistances for toroidal devices (for example, elastic flap valves) regulate the flow of breathed air, offering greater resistance to the exit of air in the expiratory phase than to the entrance of air in the inspiratory phase. Therefore, a subject who has installed this variant of the toroidal nasal device may experience a double effect: the increase in the flow of air inspired due to the separation of the tissues of the nasal structure, and the creation of expiratory positive airway pressure (EPAP). The combination of this double effect is one of the capacities that characterize the toroidal nasal device with airflow resistances against the devices of the state of the art.

For the use of a toroidal nasal device according to the present invention, the user, prior to the application of the same in the nasal cavities, changes the geometry of its toroidal structure with the closing element incorporated into the device, so that the toroidal structure acquires the characteristic form of the "Infinity" symbol (∞), comprising at least two lobes of the toroidal structure, each of which can be inserted into a subject's nasal cavity.

The user can then partially introduce the toroidal nasal device into both nasal cavities by exerting slight pressure with his hands. The toroidal nasal device may have a specific position depending on how it should be introduced into the nasal cavities, i.e., some parts of the device should be placed in the lower area of the nasal cavity, and others in the upper area. In different embodiments of the invention, certain areas of the toroidal structure of the device, with circular radial sections in smaller diameter, may be located in the upper area of the nasal cavity, and others, with larger diameters, may be located in the lower area of the nasal cavity. To clarify this position, the toroidal nasal device can have the closing element anchored permanently in the area that must be facing "downwards", before being applied by the user.

Once applied, the toroidal nasal device stabilizes itself inside the nasal cavities by means of its toroidal structure and connects both nasal cavities on the outside of the nose, putting the closing element at the bottom thereof between the two nostrils. After being installed and stabilized in the nasal cavities of the user, the device can separate tissues of the nasal anatomy and modify the nasal airflow. In the variant with airflow resistances, the device, once installed and stabilized in the nasal cavities, can modify the nasal airflow by separating the nasal tissues and simultaneously creating expiratory positive airway pressure (EPAP).

After removing the toroidal nasal device following each use, the user can unpin the closing element of the toroidal structure of the device, so that the device recovers its original ring-shape as before having been handled by the user. This allows the toroidal nasal device to keep its flexo-elastic properties over a longer time period, and also facilitates its cleaning and sterilization.

The effects generated by the toroidal nasal devices described herein, can be beneficial for the user if they are continued for a period of time sufficiently long, as for example during the sleep phase, a prolonged physical activity or similar situations.

Another object of the invention relates to a device according to any of the embodiments described in this document, for use in the treatment of respiratory diseases and/or disorders.

Another object of the invention refers to a method of configuring a nasal device according to any of the embodiments described in this document, comprising to alter the shape of the flexible toroidal structure, causing it to adopt a new form, that of the "Infinity" symbol (∞), comprising at least two lobes of said toroidal structure suitable for insertion into the subject's nasal cavities.

Below follows a description of some of the specific embodiments of toroidal nasal devices according to the invention, describing its main components as well as the process of installation and arrangement in different nasal anatomies.

### DESCRIPTION OF THE FIGURES

**Fig. 1** shows the general shape of a toroidal nasal device according to the invention, before adopting its application configuration, where its toroidal structure and its closing element are shown.
**Fig. 2** shows the device of the invention of Figure 1, where the toroidal structure is configured in its application position, forming two lobes through the closing element.
**Fig. 3** shows the device of the invention of Figure 2, where the toroidal structure is configured in its application position and lodged in the nasal cavities.
**Figs. 4a-4c** show a toroidal nasal device according to a preferred embodiment of the invention, where the toroidal structure comprises a plurality of airflow resistances. Figure 4a shows a view of the structure in its configuration prior to application, Figure 4b shows a view of the structure in its configuration of two lobes, suitable for application to the user's nasal cavities, and Figure 4c shows a view where the toroidal structure is configured in its application configuration and lodged in the nasal cavities.
**Figs. 5a-5b** show a preferred embodiment of the device of the invention, in front and profile views.
**Figs. 6a-6b** show a preferred embodiment of the device of the invention, where it has one or more areas of its geometry shaped to rest in the lower area of a subject's nasal cavities, and/or to rest in the upper area of the nasal vestibules, in front and profile views.
**Figs. 7a-7e** show different views, in elevation and profile, of closing elements for a toroidal nasal device according to the invention, where said closing elements have different forms.
**Figs. 8a-8d** show two preferred embodiments of the device of the invention, in front and profile views, where said device comprises one or more airflow resistances connected to the toroidal structure for modifying a subject's nasal airflow.

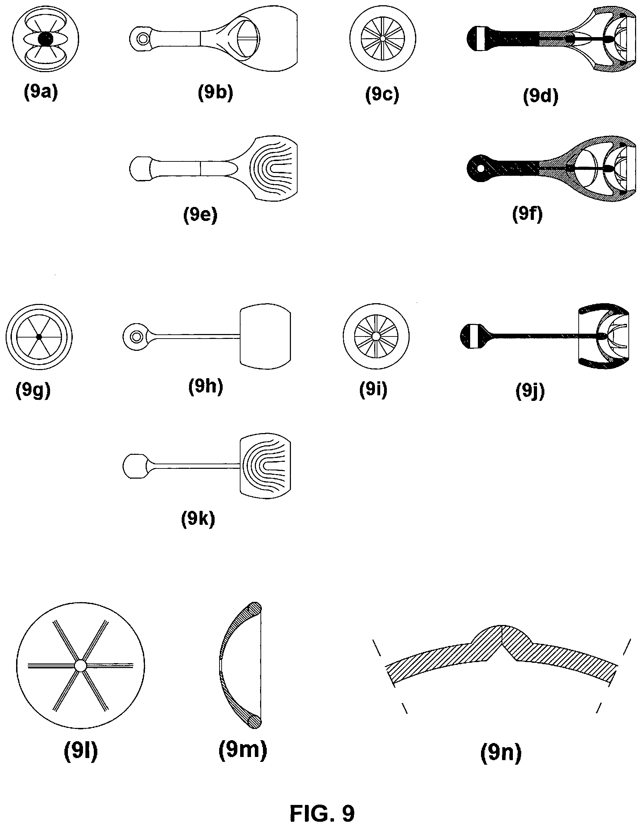

**Figs. 9a-9n** show some of the configurations that the airflow resistances can adopt according to different embodiments of the invention. In particular, Figures 9a-9f show different views (front and rear (9a, 9c) side elevations, vertical elevation (9b), front elevation (9e) and vertical (9d) and front sections (9f)) of a first embodiment of an airflow resistance, and figures 9g-9n show views (front and rear (9g, 9i) side elevations, vertical elevation (9h), front elevation (9k), vertical (9j) and front (9k) sections of the valve, front elevation of flaps (9l), profile of flaps (9m) and section of flaps (9n)) of a second embodiment of the resistance, where in both embodiments said resistance comprises a flap valve.
**Figs. 10a-10b** show two embodiments of the invention, where the toroidal structure is configured from a lineal structure that closes on itself via the closing element.
**Fig. 11** shows a 3D representation of a variant of a toroidal nasal device according to the invention, before adopting its application configuration, where its toroidal structure and its closing element are shown.
**Fig. 12** shows a 3D representation of a variant of a toroidal nasal device with airflow resistances according to the invention, before adopting its application configuration, where its toroidal structure, its closing element and the specific flap valves for this kind of device are shown.

### DETAILED DESCRIPTION OF THE INVENTION

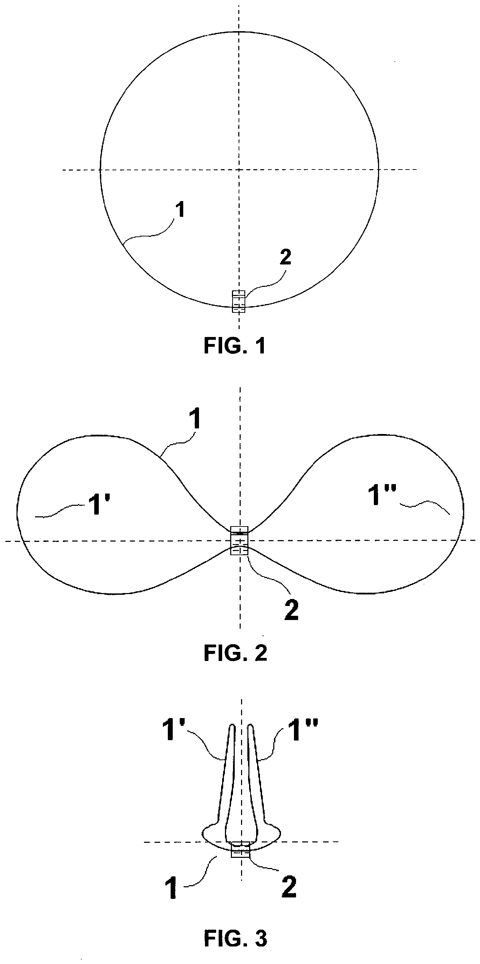

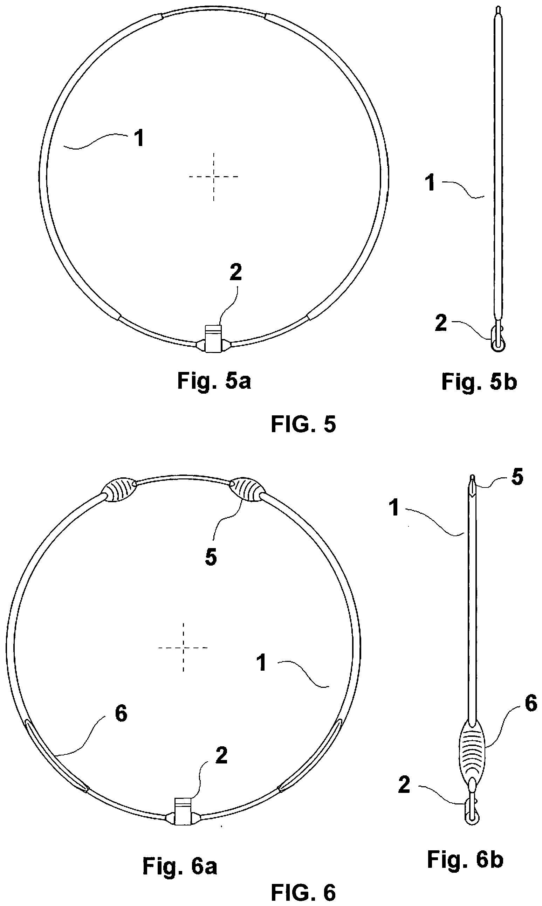

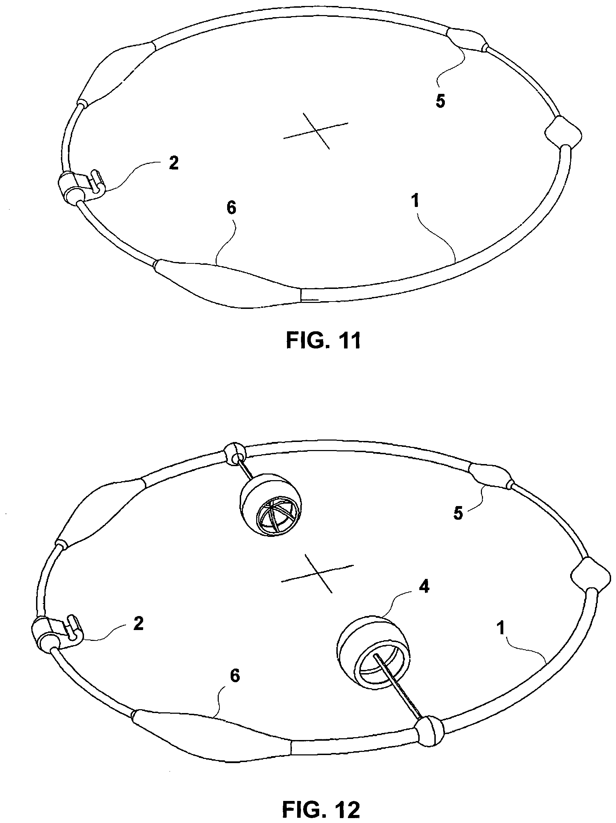

Now we proceed to describe some of the preferred and not limiting embodiments of the invention, in relation to the numerical references of the Figures 1-12 herein.

### - Main elements of the toroidal nasal device:

The toroidal structure (1) (Figure 1) is an essential element of a toroidal nasal device according to the present invention. The toroidal structure (1) possesses a geometry in the form of a toroid (ring), whose radial section may vary in shape and size, depending on the specific functional requirements of its application. These functional requirements can be of mechanical character, adaptability to the nasal anatomy or modification and control of the nasal airflow. Preferably, the toroidal structure (1) is made of flexible materials, so that it can be adapted to the different anatomies of the nasal cavities and be manipulated by the user to alter the shape of the toroidal structure (1) for placing in the nose and subsequent use.

In a preferred embodiment of the toroidal nasal device, the flexible toroidal structure (1) is connected, in a region of its geometry, to a closing element (2) configured to adapt temporarily or permanently said toroidal structure (1) to a certain shape, giving it a geometry different from the o-ring geometry and suitable for insertion into the user's nasal cavities. In Figure 2 herein it is shown how, on the basis of the flexible toroidal structure (1) of the Figure 1, it is possible to configure said structure with a form of "Infinity" symbol (∞), through the above-mentioned closing element. Said closing element (2) causes the toroidal structure (1) to adopt a new shape comprising at least two lobes (1', 1"), each of which can be inserted into a subject's nasal cavity, with the closing element (2) being arranged, after application, in the lower region of the nose between the two nostrils. In this way, when the device is inserted into the subject's nasal cavities (Figure 3), each of the lobes (1', 1") of the toroidal structure (1) presses against the inner surface of the nasal cavities, modifying the subject's nasal airflow.

Alternatively, in different variants of the toroidal nasal device, it is possible to include in one or more areas in certain parts of the toroidal structure (1), support elements (5, 6) that are configured to be located in the area of the nasal vestibule (external nasal valve) and/or in the lower internal area of the nasal cavity, that increase the contact surface between the flexible toroidal structure (1) and the nasal tissues.

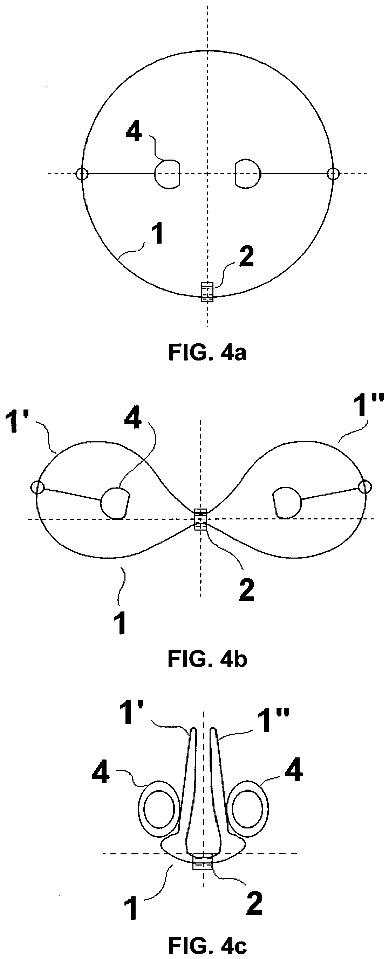

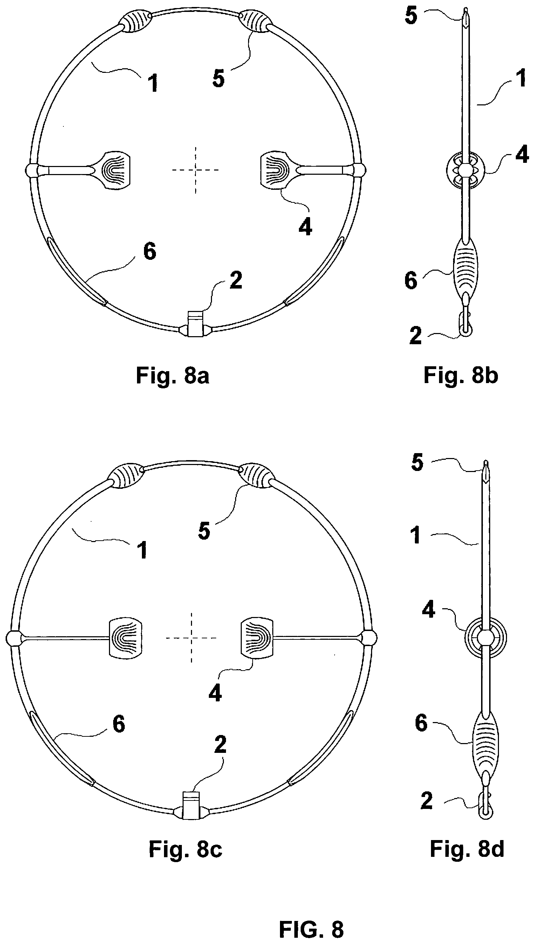

In other preferred embodiments of the invention (Figures 4a-4c and 8a-8d), the toroidal nasal device comprises one or more airflow resistances (4). These resistances (4) are designed to be used in a toroidal nasal device and depend on said toroidal structure to be located inside the nasal cavities and regulate the subject's nasal airflow.

### - Toroidal structure (1) for a toroidal nasal device:

As stated above, the toroidal structure (1) has a radial section that is preferably circular, which may vary its shape and diameter to form the anchorage area of the closing element (2); to better adapt itself to the nasal anatomy; and to have a different capacity of mechanical response (flexibility) in the areas of the structure that are located in the upper area of the nasal cavity than in areas located in the lower area of the nasal cavity.

The toroidal structure (1) of variable radial section, as all its variants, can be made of one single material (mono-material) or several, partially or completely flexible, materials (multi-material) that provide the necessary physical qualities to said structure to meet the purpose for which it is designed.

In a preferred embodiment of the invention, the toroidal structure (1) can be made of a single, hydratable, partially or totally flexible, material (mono-material), which may optionally change slightly in volume and that complies with the mechanical and comfort requirements necessary for use.

In another preferred embodiment of the invention, the toroidal structure (1) of variable radial section can be made of two materials (bimaterial), complementary to each other but with different physical characteristics, working in coherence to improve their mechanical capacities and optimize their adaptability to the nasal anatomy. In said embodiment, a first structure, made of a more rigid material and with a greater structural support, is embodied totally or partially in a second structure, made of a more flexible material (for example, with a consistency that is more suitable to be in contact with body tissues). This improves the mechanical behavior of the structure and allows a more comfortable use of the toroidal nasal device over longer periods of time.

A toroidal structure (1) for a toroidal nasal device can be formed as a continuous, unitary object; i.e., none of its parts are mechanically assembled, subsequently to their manufacture, leaving a structural joint which cuts the continuity of the material or materials with which it is made. Therefore, the outer surface of the object can be continuous and unitary.

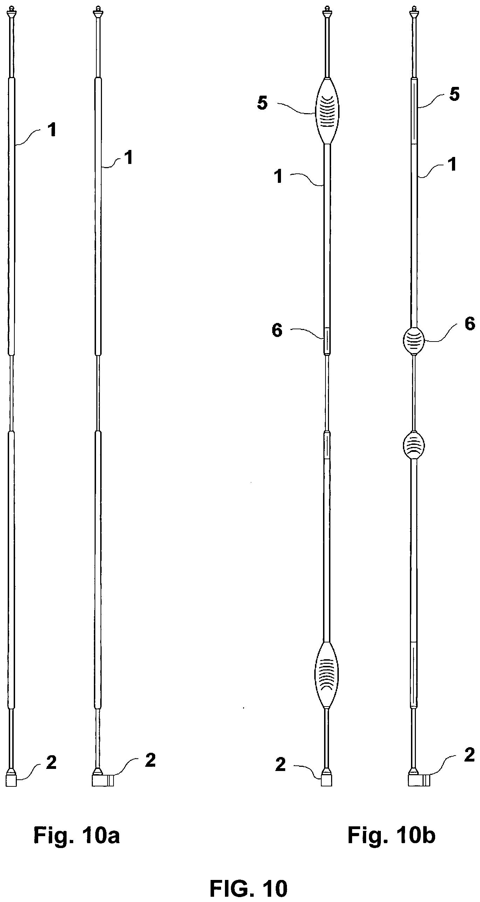

Additionally, and as shown in Figures 10a-10b, it is also possible to manufacture the toroidal structure (1) as an open and flexible structure (for example, a linear structure) that can close on itself by its ends, forming the toroidal structure of the device. Said ends can be attached, for example, in the closing element (2) of the device. This alternative may result in easy-to-make and easy-to-pack nasal devices that can be configured in toroidal form by the user himself.

The shape and dimensions of the toroidal structure (1) of variable radial section, as well as all its variants, can be modified for a better adaptation to the wide variety of existing physiognomies, provided it retains the characteristics and properties of a toroidal structure for the above-mentioned toroidal nasal device.

The texture of the outer surface of the toroidal structure (1) of variable radial section can also vary depending on the functional requirements for each of the different areas that make up its geometry. Some areas of the toroidal structure (1) can have a surface that is more or less rough, or with some kind of surface graphics, to improve their adhesion to the nasal tissues and drain more easily any mucus or fluid. For example, the texture of the areas of the toroidal structure (1) formed as support areas can simulate the design of the fingerprints of a human being.

The toroidal structure (1) of variable radial section is based on the geometry of toroids; therefore, it has a main axis of revolution on which rotates its radial section, forming its characteristic ring shape. As previously mentioned, the shape and the size of the radial section of said toroidal structure (1) varies, as it rotates around the axis of revolution, responding to functional requirements. For example, the perimeter of said radial section can be of circular, ovoid, elliptical, lobular shape and all types of regular and irregular geometries. In a preferred embodiment of the invention, most of the radial sections of the toroidal structure (1) can be circular to better adapt to the different anatomies and improve its capacity of elastic response in the three spatial directions. In other embodiments of the invention, the radial section of those areas of the toroidal structure (1) that are formed to rest inside the nasal cavity, can have an essentially elliptical shape, to increase the contact surface between said structure and the nasal tissues, thus improving the distribution of compressive stresses and the adhesive capacity. In further embodiments, the radial sections of the anchorage zones of the closing element (2) appear with a circular shape of larger diameter to better support occasional torsional and shearing stresses, and to prevent the sliding of the element along the body of the toroidal structure (1). In one of the variants shown here, the radial section of the toroidal structure (1) is modified to form one or more anchorage areas for one or more airflow resistances (4) created specifically for use in a toroidal nasal device.

In a toroidal structure (1) of variable radial section, the areas formed to fulfill more specific functions, as described herein, may vary their position and angle to the axis of revolution within the geometry of said structure. In general, the toroidal structure (1) of variable radial section can have continuity in one or more of the materials of which it is made, throughout all its radial sections, in order to improve the transmission of internal stresses (torsion, bending, compression) within the same and to enhance its mechanical response capacity using the least amount of materials.

The toroidal structure (1) of variable radial section, as all its variants, can be made of any suitable material, including but not limited to metals, plastics, rubbers, natural rubbers, ceramics, chrome and combinations thereof. Other materials can include acrylics, latex, polyethylene, polypropylene, polystyrene, polyvinyl chloride, polyvinylidene chloride, polyvinyl acetate, polyacrylate, styrene-butadiene copolymer, chlorinated polyethylene, polyvinylidene fluoride, ethylene vinyl acetate copolymer, acrylate chloride-vinyl acetate-ethylene-vinyl copolymer, ethylene acetate-vinyl acrylate copolymer, vinyl chloride-ethylene acetate-vinyl copolymer, nylon, acrylonitrile-butadiene copolymer, polyacrylonitrile, polyvinyl chloride, polychloroprene, polybutadiene, thermoplastic polyimide, polyacetal, polyphenylene sulphide, polycarbonate, thermoplastic polyurethane, thermoplastic resins, thermosetting resins, natural rubbers, synthetic rubbers (such as chloroprene rubber, styrene butadiene rubber, nitrile-butadiene rubber, and ethylene-propylene-diene terpolymer copolymer, silicone rubbers, fluorinated rubbers and acrylic rubbers), elastomers (such as the soft urethane or water-expanded polyurethane), and thermosetting resins (such as hard urethane, phenolic resins and melamine resins).

In a preferred embodiment of the invention, biocompatible materials can be used, especially in the areas of the toroidal structure (1) in direct contact with the user's body tissues. In addition to some of the materials described above, biocompatible materials can also include a biocompatible polymer and/or elastomer. Suitable biocompatible polymers may include materials such as: vinyl acetate homopolymer and copolymers (such as ethylene vinyl acetate copolymer and polyvinyl chloride copolymer), alkylstyrene copolymers, acrylate homopolymer and copolymers (such as polypropylene, polymethylmethacrylate, poly-methacrylate, polymethacrylate, ethylene glycol dimethacrylate, ethylene dimethacrylate, siloxane methacrylate and hydroxymethyl methacrylate, and the like), polyvinylpyrrolidone, 2-pyrrolidone, polyacrylonitrile butadiene, polyamides, fluoropolymers (such as polytetrafluoroethylene, fluorinated methacrylate siloxane and polyvinyl fluoride), styrene acrylonitrile homopolymer and copolymers, cellulose acetate, acrylonitrile butadiene styrene homopolymer and copolymers, polymethylpentene, polysulfones polyimides, polyisobutylene, polymethylstyrene and other similar compounds belonging to the state of the art.

In the present invention, the use of materials that are particularly flexible and elastic is advantageous, as these can be used in the same way as biocompatible and sterilizable materials, such as for example: medical-use plastics (such as acrylonitrile butadiene styrene (ABS), latex, polypropylene, polycarbonate and polyetheretherketone), silicone, polyisoprene (PI) and other cross-linked elastomers or thermoplastic elastomers (TPE).

Other materials such as Teflon, Mylar, PFA, LDPE, Hytrel, HDPE and polyester can also be used in some parts of the toroidal nasal device of the invention.

### - Closing element (2):

The closing element (2) is another of the essential elements comprising the toroidal nasal device of the invention. The closing element (2) is used to modify the shape of the toroidal nasal device, in order to enhance its mechanical response capacity, improve its adaptability to the different nasal physiognomies; and facilitate the application of the device by the user.

In one of its variants, the closing element (2) can be attached at the bottom of the nose, between the two nostrils, to collaborate, in a complementary manner, with the toroidal structure (1) in the stabilization of the device within the user's nasal anatomy.

The closing element (2) can modify the shape of the device, anchored in two or more points of its toroidal structure (1) to modify its ring shape and temporarily prevent the structure from returning to its original position after handling. In the examples described herein, the closing element (2) is anchored to two opposite points of the geometry of the toroidal structure (1), bringing them closer to the axis of revolution of the same, and resulting in a characteristic form of the "Infinity" symbol (∞) that improves the capabilities of the above-mentioned device.

The closing element (2) of a toroidal nasal device typically comprises two or more areas of its geometry formed to be anchored in the toroidal structure (1) of the device; and in one of its variants, one of its sides formed to accommodate a replaceable adhesive element that can attach the closing element (2) to the bottom of the user's nose. In another of its variants, one of its sides is formed as a structure that can attach the closing element (2) to the bottom of the user's nose.

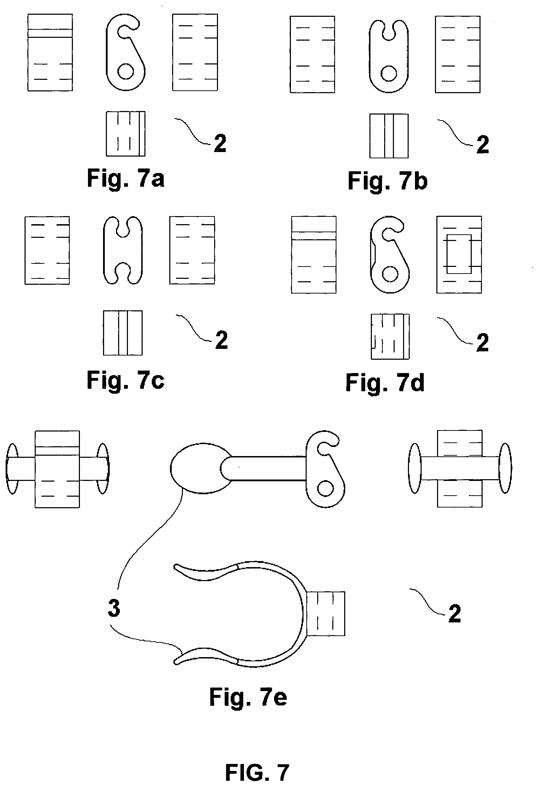

The shape of a closing element (2) for a toroidal nasal device may vary depending on the functional requirements and the material or materials from which it is made. Generally, shapes with geometric and constructive simplicity are preferable, such as for example epsilon- (ε), delta- (δ), beta- (β), omega- (Ω), theta-shape (θ), etc. Some examples of these forms are shown in Figure 7 of the present document.

In different variants of the toroidal nasal device (Figure 7e), the closing element (2) can be connected, in one or more areas of its geometry, to one or more support elements (3) configured to be located preferably at the bottom of the nasal septum outside of the nose and which collaborate with the toroidal structure (1) for the stabilization of the device. The example in Figure 7e shows a closing element (2) with a clip-type support element (3), designed to be placed between the user's nostrils.

The closing element (2) of a toroidal nasal device can be anchored temporarily or permanently to the toroidal structure (1). For example, the closing element (2) can be anchored permanently to a point of the geometry of the toroidal structure (1) by means of a ring-shaped anchorage, and can be anchored to another point of the toroidal structure (1) by means of a clip- or clamp-shaped anchorage.

In addition, the closing element (2) of a toroidal nasal device can use a patellar-type anchorage, i.e., an anchor designed to allow the free rotation of the o-ring body inside the same (hinge) in order not to create additional torsional stresses on the radial section of the toroidal structure (1) that could negatively impact the mechanical behavior and the adaptive capacity of the same.

In a preferred embodiment of the closing element (2) of a toroidal nasal device according to the invention, said element may have two areas of its geometry formed as an anchoring system in the form of a clip, clamp or ring, which can vary their position or orientation within the same based on functional requirements. Figure 7 shows various shapes of a closing element (2) for a toroidal nasal device, with two areas of its geometry formed as patellar anchorages. One of these areas has a ring shape to be anchored permanently to the toroidal structure (1) of the toroidal nasal device, and another has a clip or clamp shape to be anchored temporarily in the toroidal structure (1) during the periods of use of the device.

In a preferred embodiment of the invention, one of the faces of the closing element (2) of the toroidal nasal device is made to accommodate a replaceable adhesive element (for example, a liquid, gel, paste, strips, fabric, etc.), in order to fix or attach said closing element (2) at the bottom of the user's nose, between the two nostrils.

Materials or adhesive elements used for this variant of the closing element (2) for a toroidal nasal device must be biocompatible and suitable for use without any risk for the user. Adhesives for medical use are especially desirable (hot melt or acrylic).

A closing element (2) of a toroidal nasal device can be made of a single material (mono-material) or several materials (multi-material) that comply with the requirements for proper operation and ensure the durability of the device. For example, any of the materials mentioned previously in this publication may be used, provided that it complies with the requirements of safety and durability. Flexible and hard plastics in particular can be used for the closing element (2) of a toroidal nasal device. Depending on its configuration, the closing element (2) may be made of a thermoplastic plastic or a cross-linked or thermoplastic elastomer (e.g. polypropylene).

### - Airflow resistances (4) for a toroidal nasal device:

The toroidal nasal device with airflow resistances (4) is one of the variants of the toroidal nasal device, which incorporates one or more airflow resistances (4), created for this type of device, which regulate the subject's nasal airflow (Figures 8a-8d of the present document).

An airflow resistance (4) for a toroidal nasal device is characterized by essentially operating by "traction", and by depending on the toroidal structure (1) of the device and the arm that connects the two, to be able to function properly and to be located inside a subject's nasal cavities.

An airflow resistance (4) for a toroidal nasal device can be positioned inside the nasal cavity, in direct contact with the flow of breathed air, making part of the latter pass through, or around, this resistance, in order to regulate and control said flow of air. Therefore, said airflow resistance (4) regulates the level of resistance to the flow of air, the flow rate and pressure differentials in the airway. Preferably, the airflow resistances (4) offer greater resistance to the flow of air in one direction than in the opposite direction (for example, greater resistance to air exiting during exhalation than to air entering during inspiration). In this case, the flow resistance may be capable of generating an expiratory positive pressure inside the airway (EPAP), which can vary in levels depending on various factors, such as the dimension of the valve, the design of the same and the user's anatomy itself. The ideal range of pressure levels that could be generated by the flow resistance can vary from 0.5 to 25 cm of H2O, measured for a flow rate of 100 ml/s.

Preferably, the airflow resistances (4), used for toroidal nasal devices of the invention, are resistances of the valve- or flap-type, or the like. In Figures 9a-9n of the present document, examples of such resistances are shown. In particular, the figures 9a-9f show different views of a first embodiment of an airflow resistance, and figures 9g-9n show a second embodiment thereof, where in both embodiments such resistance comprises a flap valve.

A flap valve (Figures 9a-9k) for a toroidal nasal device has a series of particularities compared with other flap valves designed for other nasal devices. These particularities are, among others, the lack of escape routes of air, both in the valve body and the flaps of the valve, when the valve is closed or blocked; its dependency on and configuration around a structural axis, which serves it as a support and which anchors it to the toroidal structure (1) of the device; and its high deformability and flexibility due to its curved shapes configured around said structural central axis and the geometric simplicity of the valve body without air escape routes. The absence of air escape routes in a flap valve for a toroidal nasal device, is due to its location inside the anatomy of the nasal cavity, where the valve does not occupy the whole of the flow section of the nostril and air can flow around the valve body when the valve is closed, without being obstructed by the nose hairs (vibrissae) or other tissues. This allows a minimum airflow for proper operation and breathing.

A flap valve for a toroidal nasal device may comprise a more or less flexible valve body, that protects the flaps located inside, facilitating their correct operation; one or more flexible flaps (Figures 9l-9n) that can be configured to be opened in one direction of flow and closed totally or partially in the opposite direction, even when the flow does not exist; an internal structure, included in the valve body, that serves as a support for the flaps and for the valve body itself; and a structural axis that supports the rest of the elements that comprise the valve and which is anchored, at one end, to the toroidal structure (1) of the toroidal nasal device.

The elements comprising said flap valve for a toroidal nasal device may be formed as a continuous and single element, which is embodied in the geometry of the toroidal structure (1) of the device.

A flap valve for a toroidal nasal device may be based, for example, on spherical or parabolic geometries. In one possible embodiment of the invention (Figures 9a-9f), the body and the valve flaps can be formed as a single element with intersecting ovoid and sphere shapes, leaving a passage of air through them. Said element is supported by a hemispherical structure lodged in its interior and a structural axis of circular section, which crosses it longitudinally and anchors them to the toroidal structure of the device.

In another possible embodiment of the invention (Figures 9g-9k), the body, the semispherical structure and the structural axis of the valve may be formed as a single element with intersecting ovoid and sphere shapes, being anchored to the toroidal structure and leaving a passage of air through them. Said element serves as a support for the element of the valve flaps (Figures 9l-9n) formed as a spherical shell with variable thickness that unifies said flaps.

The structural axis of a flap valve for a toroidal nasal device is the element that provides structural support to the elements of the valve, by attaching them to the toroidal structure (1) of the device. Said axis allows the valve to operate correctly and stabilize itself at the desired place, behind or in front of the internal nasal valve, in order to regulate and modify the nasal airflow. The anchoring of the structural axis of the valve on the toroidal structure (1) can be fixed or of a patellar type.

Said resulting shape of the intersection of spherical forms may be especially suitable in embodiments with flexible materials, thanks to its stability at moments of maximum pressure during exhalation (closed or semi-closed valve) and its ability to adapt to the different physiognomies of the nasal structure.

For the toroidal nasal device of the invention, particularly desirable for the formation of the airflow resistances (4) are spherical, hyperbolic, parabolic, spiral geometries, and all types of organic geometries.

In a flap valve for a toroidal nasal device according to the invention, the shape and the number of flaps (Figures 9l-9n) may vary depending on the specific needs for its optimum performance according to the user typology. As an example of a possible embodiment, the flaps can take the form of a spherical shell, and their number may vary between three and nine. In other embodiments of the invention, the flaps of the valve can be designed so that the geometry of their edges (curved rib with different support angles among them) allows them to absorb deformations of the valve body without substantially impacting the operation of the same. The flaps of the mentioned embodiments can be made by cuts in pre-made forms, to obtain the desired result in the regulation of the airflow.

In different embodiments of the invention, the surface texture of the airflow resistance (4) of a toroidal nasal device can vary, in order to improve its adhesive capacity or comfort. For example, the surface can be more or less rough and have some kind of graphics or specific design in some areas with more precise requirements, such as in areas of greater contact with body tissue.

The shapes of the airflow resistances (4) for a toroidal nasal device can be very varied, spherical, elliptical, ovoid, conical, in diatom shape, in irregular shapes, etc. The realization of the airflow resistances (4) for a toroidal nasal device can have any shape or geometry necessary to properly fulfill the function for which it is designed. The dimension, shape, angle and position of the airflow resistances (4) in a toroidal structure (1) for a toroidal nasal device according to the invention may vary depending on the adaptability to the different existing physiognomies and the nature of the materials with which they are carried out. In different preferred embodiments of the invention, the airflow resistance (4) for a toroidal nasal device can be made with the same material(s) as the toroidal structure (1) of variable radial section that supports it or the closing element (2) mentioned above. Also, it can be made of materials arranged in layers or in alloys.

### - Manufacture of the toroidal nasal device:

The different elements that comprise the toroidal nasal device can be made using different industrial processes (immersion, injection, centrifugal casting, turning, thermoforming, 3D printing, etc.), depending on the nature of the material to be used, the complexity of its geometry, and the functional requirements. For example, the toroidal structure (1) of the toroidal nasal device and all its variants can be configured to be a continuous and single element, for which the industrial techniques of molding by immersion, injection or 3D printing may be the most appropriate to said objective. Also, the different elements of the airflow resistance (4) for a toroidal nasal device can be made with these methods.

For example, for the manufacture of the closing element (2) of the toroidal nasal device, given the characteristics mentioned above, the techniques of molding by injection or thermoforming may be most appropriate. However, any industrial procedures can be used that are necessary to attribute to the elements of the toroidal nasal device the functional characteristics required.

If the elements are made by means of a procedure of immersion in dispersions, solutions, or masses in a viscous state (e.g. nets or liquefied polymers), the different thicknesses of the material layers can be carried out by immersion molds with varied geometries. For example, the reductions or thickening of section in the material can be achieved by immersion of molds with edges that have small radii of curvature. The surface tension of the material of the dissolution, together with the geometry and the temperature of the mold used, can generate material sections that are variable in curvature and thickness. These molds can be designed to be "lost" molds, i.e. can become permanently imbued in the material of the dissolution after application, becoming an internal substructure of the toroidal nasal device. Where the manufacture of the elements is carried out through methods of molding by injection or molding by compression, the reductions, the thickening and the different surface textures can be made by machining in the mold, both in the male and the female.

In some embodiments of the single body element and flaps for an airflow resistance (4) of a toroidal nasal device, the process may require up to five molds, three males and two females. Said molds determine the internal and external geometry of the element to be manufactured so that it allows the removal of the same once the element has been injected.

Another method that can be used for manufacturing the elements of the toroidal nasal device of the invention is the printing of polymers by means of a 3D printer.

In some preferred embodiments of the invention described, the elements of the flap valve for the toroidal nasal device can be attached to each other by means of chemical or ultrasonic welding.

The flaps of an airflow resistance (4) for a toroidal nasal device may be made by cuts, with blades or laser light, on the previously shaped forms.

The permanent anchor of the closing element (2) on the toroidal structure (1) of the toroidal nasal device can be done through the application of a force or using an ultrasonic welding on the element, so that it is anchored or shaped around the geometry of the toroidal structure (1).

In one of its variants, the closing element (2) of a toroidal nasal device has one of its sides adapted to accommodate a replaceable adhesive element that can be applied prior to the use of the device and removed at the end of the period of use thereof. For example, the adhesive element can be a liquid, gel, paste, which is applied with an applicator, on the closing element (2) before use and is removed, by means of a washing, at the end of the period of use. Also, the adhesive element may include a replaceable double-sided adhesive strip, which can be applied, on one side, over the closing element (2) of the toroidal nasal device and, on the other side, against the bottom of the user's nose, between the nostrils.

### - Examples of preferred embodiments of the invention:

In different preferred embodiments, the dimensions of the overall diameter of the variable section toroidal structure (1) of the invention may vary between 30 and 90 mm, and the external diameter of the airflow resistance (4) may vary between 16 and 6 mm.

Figures 1-4 of the present document show general schemes of the device of the invention, with its main positions that must be configured by a user for application. Figures 1-2 show how the user, prior to the application in the nasal cavities and with their own hands, starts with an unmodified toroidal structure (1) (Figure 1), for subsequently configuring said structure (1) in an application position (Figure 2), by means of the closing element (2) incorporated into the device. This configuration gives the toroidal nasal device a characteristic shape of two lobes forming an "Infinity" symbol (∞), which facilitates the application of the same by the user and enhances the above-mentioned capabilities of the device. Figure 3 shows a toroidal nasal device in front view, once it has been applied in the user's nasal cavities, where the closing element can comprise, in addition, a replaceable adhesive support element or a support structure in clip form (3) to attach said element at the bottom of the nose. Figures 4a-4c show arrangements similar to those of the Figures 1-3, but for those embodiments of the invention comprising airflow resistances (4).

Once the device is configured in its application form of two lobes (Figures 2, 3 and 4b-4c), the user must position the toroidal nasal device, taking as reference the permanent anchor of the closing element (2), before applying it in the nasal cavities by exerting slight pressure with his fingers. The application of the device can be simultaneously in both nasal cavities or alternatively, first in one cavity and then in the other. The toroidal nasal device must be installed with the toroidal structure (1) partially located in both nasal cavities and connecting them on the outside of the nose, and with the closing element (2) located between the two nostrils and aligned with the nasal septum on the outside of the nose.

In some variants of the toroidal nasal device, the closing element (2) can be attached at the bottom of the nose, by means of a replaceable adhesive element.

In general, in order to assist the user in the installation, the toroidal nasal device can be applied in front of a mirror (but it can also be applied without it). Once applied, the user will feel how the toroidal nasal device stabilizes itself in the anatomy of the nasal cavities by means of its toroidal structure (1), acting directly on the nasal airflow.

The user will have to verify that the device is installed and stabilized in the nasal cavities in a manner that is comfortable. The user should also verify that the device is operating correctly, as it should lead him to feel an improvement in the fluidity of the airflow both during inspiration and expiration. In the variant of the toroidal nasal device with airflow resistances (4), the user should feel an improvement in fluidity of the airflow during inspiration and a resistance to the outflow during exhalation that will create positive pressure inside the airway.

After using the toroidal nasal device, the user may remove this device by pulling slightly on its toroidal structure (1) or on its closing element (2), until it comes completely out of the nostrils. In the variant in which the closing element (2) is attached between the two nostrils, the user must remove said closing element (2) before extracting the toroidal nasal device. Similarly, in those embodiments of the invention using a closing element in clip form (3), the user should first remove the same from the nasal septum before extracting the toroidal nasal device.

After removing the device, the user should unpin, with his fingers, the closing element (2) of the device of the toroidal structure, allowing it to recover its original ring shape. This extends the useful life of the device and facilitates cleaning and sterilizing before a new application, in case of using a toroidal nasal device with reusable or non-disposable elements (for example, the toroidal structure (1) and/or the closing element (2)). It is also possible, in other embodiments of the invention, to use the device in a disposable manner, as concerns the toroidal structure (1) and/or the closing element (2). In such embodiments, the different elements of the toroidal nasal device may be optionally made of biodegradable materials.

## Claims

1. Nasal device adapted to modifying a subject's nasal airflow, comprising:
- a flexible toroidal structure (1), adapted to be introduced at least partially into the subject's nasal cavities;
- a closing element (2) connected to the toroidal structure (1), equipped with means to alter the shape of said toroidal structure (1), causing it to adopt a new shape comprising at least two lobes (1', 1"), each of which can be inserted into a subject's nasal cavity; wherein the closing element (2) is adapted to be arranged in the lower external region of the nose between the two nostrils so that, when the flexible toroidal structure (1) is inserted into the subject's nasal cavities, each of the lobes (1', 1") of the toroidal structure (1) presses against an inner surface of said nasal cavities.

2. Device according to the preceding claim, where the closing element (2) comprises one or more areas of its geometry shaped in the form of a clip, clamp or ring configured to be anchored in a patellar manner to the toroidal structure (1), thereby allowing the free spinning of the toroidal structure (1) body inside the closing element (2); and to modify the shape of said toroidal structure (1), adopting the form of an infinity symbol.

3. Device according to any of the preceding claims, where the toroidal structure (1) comprises one or more support elements (5, 6) formed to rest in the lower area of a subject's nasal cavities, and/or to rest in the upper area of the nasal vestibules of the same.

4. Device according to any of the preceding claims, where the toroidal structure (1) is configured from a linear structure that closes on itself by means of the closing element (2).

5. Device according to any of the preceding claims that further comprises one or more airflow resistances (4) connected to the toroidal structure (1) for modifying a subject's nasal airflow.

6. Device according to the preceding claim, where the airflow resistances (4) comprise flap valves.

7. Device according to any of the preceding claims, where the closing element (2) further comprises an area of its geometry shaped in clip form (3), configured to be fixed to the lower external region of the nose between the two nostrils.

8. Device according to any of the preceding claims, where the closing element (2) further comprises an area of its geometry shaped to accommodate a replaceable adhesive element.

9. Device according to any of the preceding claims, further comprising a replaceable adhesive element configured to be adhered on one side to the closing element (2) and, on the other side, to the lower external region of the nose between the two nostrils.

10. Device according to any of the preceding claims, wherein:
- the toroidal structure (1) is made of a single partially or totally flexible, hydratable material; or
- the toroidal structure (1) is made with at least two materials of different physical characteristics, where a first material is more rigid than a second material, and forms a first structure embodied totally or partially in a second structure of the second, more flexible material.

11. Device according to any of previous claims, wherein certain areas of the toroidal structure (1) have circular radial sections with smaller diameter, while others have circular radial sections with larger diameter.

12. Device according to any of the preceding claims, comprising a biocompatible and/or biodegradable material.

13. Device according to any of the preceding claims, where the toroidal structure (1) and/or the closing element (2) are reusable.

14. Device according to any of the preceding claims for use in the treatment of respiratory diseases and/or disorders.

15. Method for configuring a nasal device according to any of the claims 1-13, comprising: the alteration of the shape of the flexible toroidal structure (1) by means of a closing element (2) incorporated into the device, making that toroidal structure (1) adopt a new shape comprising at least two lobes (1', 1") suitable for insertion into a subject's nasal cavities; and/or undoing the alteration of the shape of the toroidal structure (1) of two lobes (1', 1 "), recovering its toroidal shape.

## Patentansprüche

1. Nasenvorrichtung, die zum Modifizieren des Nasenluftstroms eines Subjekts ausgelegt ist, umfassend:
- eine flexible toroidale Struktur (1), die ausgelegt ist, mindestens teilweise in die Nasenhöhlen eines Subjekts eingeführt zu werden;
- ein Schließelement (2), das mit der toroidalen Struktur (1) verbunden ist, die mit Mitteln zum Verändern der Form der toroidalen Struktur (1) ausgestattet ist, die ausgelegt ist, eine neue Form anzunehmen, die mindestens zwei Ausbuchtungen (1', 1 ") umfasst, die jeweils in die Nasenhöhle eines Subjekts eingesetzt werden können;
wobei das Schließelement (2) ausgelegt ist, in dem unteren externen Bereich der Nase zwischen den zwei Nasenflügeln angeordnet zu werden, sodass, wenn die flexible toroidale Struktur (1) in die Nasenhöhlen des Subjekts eingesetzt wird, jede der Ausbuchtungen (1', 1 ") der toroidalen Struktur (1) gegen eine innere Oberfläche der Nasenhöhlen presst.

2. Vorrichtung nach dem vorhergehenden Anspruch, wobei das Schließelement (2) einen oder mehrere Bereiche umfasst, deren Geometrie in Form eines Clips, einer Klemme oder eines Rings geformt ist, der konfiguriert ist, auf eine patellare Weise an der toroidalen Struktur (1) verankert zu werden, sodass die freie Drehung des Körpers der toroidalen Struktur (1) im Inneren des Schließelements (2) ermöglicht wird; und um die Form der toroidalen Struktur (1) zu modifizieren, welche die Form eines Unendlichkeitssymbols annimmt.

3. Vorrichtung nach einem der vorhergehenden Ansprüche, wobei die toroidale Struktur (1) ein oder mehrere Stützelemente (5, 6) umfasst, die ausgebildet sind, um im unteren Bereich der Nasenhöhlen eines Subjekts aufzuliegen und/oder im oberen Bereich der Nasenvorhöfe derselben aufzuliegen.

4. Vorrichtung nach einem der vorhergehenden Ansprüche, wobei die toroidale Struktur (1) aus einer linearen Struktur konfiguriert ist, die sich mittels des Schließelements (2) von selbst schließt.

5. Vorrichtung nach einem der vorhergehenden Ansprüche, die ferner einen oder mehrere Luftströmungswiderstände (4) umfasst, die mit der toroidalen Struktur (1) verbunden sind, um den Nasenluftstrom eines Subjekts zu modifizieren.

6. Vorrichtung nach dem vorhergehenden Anspruch, wobei die Luftströmungswiderstände (4) Klappventile umfassen.

7. Vorrichtung nach einem der vorhergehenden Ansprüche, wobei das Schließelement (2) ferner einen Bereich umfasst, dessen Geometrie in Clipform (3) geformt ist, die konfiguriert ist, an dem unteren externen Bereich der Nase zwischen den zwei Nasenflügeln befestigt zu werden.

8. Vorrichtung nach einem der vorhergehenden Ansprüche, wobei das Schließelement (2) ferner einen Bereich umfasst, dessen Geometrie zur Unterbringung eines austauschbaren Klebeelements geformt ist.

9. Vorrichtung nach einem der vorhergehenden Ansprüche, ferner umfassend ein austauschbares Klebeelement, das konfiguriert ist, auf einer Seite an das Schließelement (2) geklebt zu werden und auf der anderen Seite an den unteren externen Bereich der Nase zwischen den zwei Nasenflügeln geklebt zu werden.

10. Vorrichtung nach einem der vorhergehenden Ansprüche, wobei:
- die toroidale Struktur (1) aus einem einzigen teilweise oder vollständig flexiblen, hydratisierbaren Material hergestellt ist; oder
- die toroidale Struktur (1) mit mindestens zwei Materialien von unterschiedlichen physikalischen Eigenschaften hergestellt ist, wobei ein erstes Material steifer als ein zweites Material ist und eine erste Struktur bildet, die in einer zweiten Struktur des zweiten, flexibleren Materials vollständig oder teilweise eingebettet ist.

11. Vorrichtung nach einem der vorhergehenden Ansprüche, wobei bestimmte Bereiche der toroidalen Struktur (1) kreisförmige radiale Abschnitte mit kleinerem Durchmesser aufweisen, während andere kreisförmige radiale Abschnitte mit größerem Durchmesser aufweisen.

12. Vorrichtung nach einem der vorhergehenden Ansprüche, umfassend ein biokompatibles und/oder biologisch abbaubares Material.

13. Vorrichtung nach einem der vorhergehenden Ansprüche, wobei die toroidale Struktur (1) und/oder das Schließelement (2) wiederverwendbar sind.

14. Vorrichtung nach einem der vorhergehenden Ansprüche zur Verwendung bei der Behandlung von Atemwegserkrankungen und/oder -störungen.

15. Verfahren zum Konfigurieren einer Nasenvorrichtung nach einem der Ansprüche 1 bis 13, umfassend: die Veränderung der Form der flexiblen toroidalen Struktur (1) mittels eines Schließelements (2), das in die Vorrichtung integriert ist, Veranlassen, dass die toroidale Struktur (1) eine neue Form annimmt, die mindestens zwei Ausbuchtungen (1', 1 ") umfasst, die zur Einsetzung in die Nasenhöhlen eines Subjekts geeignet sind; und/oder Rückgängigmachen der Veränderung der Form der toroidalen Struktur (1) von zwei Ausbuchtungen (1', 1 "), sodass sie ihre toroidale Struktur wiedererlangt.

## Revendications

1. Dispositif nasal adapté pour modifier le flux d'air nasal d'un sujet, comprenant :
- une structure toroïdale flexible (1), adaptée pour être introduite, au moins partiellement, dans les cavités nasales du sujet ;
- un élément de fermeture (2) relié à la structure toroïdale (1), équipé de moyens pour modifier la forme de ladite structure toroïdale (1), lui permettant d'adopter une nouvelle forme comprenant au moins deux lobes (1', 1 "), chacun pouvant être inséré dans la cavité nasale d'un sujet ;
dans lequel l'élément de fermeture (2) est adapté pour être agencé dans la région externe inférieure du nez entre les deux narines de sorte que, lorsque la structure toroïdale flexible (1) est insérée dans les cavités nasales du sujet, chacun des lobes (1', 1 ") de la structure toroïdale (1) presse contre une surface interne des dites cavités nasales.

2. Dispositif selon la revendication précédente, dans lequel l'élément de fermeture (2) comprend une ou plusieurs zones de sa géométrie sous forme d'une pince, d'un crochet ou d'un anneau configuré pour être ancré de manière rotulienne à la structure toroïdale (1) permettant ainsi le filage libre du corps de la structure toroïdale (1) dans l'élément de fermeture (2); et de modifier la forme de ladite structure toroïdale (1), en adoptant la forme d'un symbole de l'infini.

3. Dispositif selon l'une quelconque des revendications précédentes, dans lequel la structure toroïdale (1) comprend un ou plusieurs éléments de support (5, 6) formés pour s'appuyer dans la zone inférieure des cavités nasales d'un sujet et/ou pour s'appuyer dans la zone supérieure des vestibules nasaux de la même.

4. Dispositif selon l'une quelconque des revendications précédentes, dans lequel la structure toroïdale (1) est configurée à partir d'une structure linéaire qui se ferme sur elle-même, au moyen de l'élément de fermeture (2).

5. Dispositif selon l'une quelconque des revendications précédentes, qui comprend en outre une ou plusieurs résistances d'écoulement d'air (4) reliées à la structure toroïdale (1) pour modifier le flux d'air nasal d'un sujet.

6. Dispositif selon la revendication précédente, dans lequel les résistances d'écoulement d'air (4) comprennent des soupapes à clapet.

7. Dispositif selon l'une quelconque des revendications précédentes, dans lequel l'élément de fermeture (2) comprend en outre une zone de sa géométrie sous forme de pince (3), configurée pour être fixée à la région externe inférieure du nez, entre les deux narines.

8. Dispositif selon l'une quelconque des revendications précédentes, dans lequel l'élément de fermeture (2) comprend en outre une zone de sa géométrie formée pour recevoir un élément adhésif remplaçable.

9. Dispositif selon l'une quelconque des revendications précédentes, comprenant en outre un élément adhésif remplaçable configuré pour être collé d'un côté à l'élément de fermeture (2) et, de l'autre côté, à la région externe inférieure du nez, entre les deux narines.

10. Procédé selon l'une quelconque des revendications précédentes, dans lequel :
- la structure toroïdale (1) est constituée d'un seul matériau partiellement ou totalement flexible, hydratable ; ou
- la structure toroïdale (1) est réalisée avec, au moins, deux matériaux de caractéristiques physiques différentes, où un premier matériau est plus rigide qu'un second matériau et forme une première structure intégrée, totalement ou partiellement, dans une seconde structure du second matériau plus flexible.

11. Dispositif selon l'une quelconque des revendications précédentes, dans lequel certaines zones de la structure toroïdale (1) ont des sections radiales circulaires de plus petit diamètre, tandis que d'autres ont des sections radiales circulaires de plus grand diamètre.

12. Dispositif selon l'une quelconque des revendications précédentes, comprenant un matériau biocompatible et/ou biodégradable.

13. Dispositif selon l'une quelconque des revendications précédentes, dans lequel la structure toroïdale (1) et/ou l'élément de fermeture (2) sont réutilisables.

14. Dispositif selon l'une quelconque des revendications précédentes pour son utilisation dans le traitement de maladies et/ou troubles respiratoires.

15. Procédé de configuration d'un dispositif nasal, selon l'une quelconque des revendications 1 à 13, comprenant : l'altération de la forme de la structure toroïdale flexible (1) au moyen d'un élément de fermeture (2) incorporé dans le dispositif, permettant à cette structure toroïdale (1) d'adopter une nouvelle forme comprenant, au moins, deux lobes (1', 1 ") aptes à être insérés dans les cavités nasales d'un sujet ; et/ou l'annulation de l'altération de la forme de la structure toroïdale (1) de deux lobes (1', 1 "), en récupérant sa forme toroïdale.
